# EUROPEAN PATENT APPLICATION

(11) **EP 3 922 283 A1**
(43) Date of publication of application: **15.12.2021**
(21) Application number: 21157458.7
(22) Date of filing: 16.02.2021
(51) Int. Cl.: A61M 1/36

(54) **BLOOD COLLECTION DEVICE FOR AN EXTRACORPOREAL BLOOD CIRCUIT**

(30) Priority: 18.02.2020 IT 202000003317
(71) Applicant: Eurosets S.r.l., 41036 Medolla (MO) (IT)
(72) Inventor: FONTANILI, Paolo, 41036 Medolla (MO) (IT); GHELLI, Nicola, 41036 Medolla (MO) (IT); PETRALIA, Antonio, 41036 Medolla (MO) (IT)
(74) Representative: Zoli, Filippo

(57) **Abstract**

The blood collection device (1) for an extracorporeal blood circuit comprises:
- at least one bag (2) made of a flexible material defining a containment volume (3) of the blood, the containment volume (3) being delimited by a plurality of perimeter edges (3a,3b,3c);
- at least one inlet duct (4) of the blood associated with the bag (2) and defining an inlet port (4a) communicating with the containment volume (3);
- at least one outlet duct (5) of the blood associated with the bag (2) and defining an outlet port (5a) of the blood communicating with the containment volume (3);
- at least one exhaust duct (6) of the air defining an exhaust port (6a) communicating with the containment volume (3);
where the inlet duct (4) and the outlet duct (5) are associated with the bag (2), respectively, at a first zone (14) and at a second zone (15) defined in its lower portion and the exhaust duct (6) is associated with the bag (2) at a third zone (16) defined in its upper portion;
where the exhaust duct (6) is substantially superimposed on the inlet duct (4) and where the containment volume (3) has a maximum section, measured transversely to the longitudinal axis of the ducts (4, 5, 6), in the proximity of the inlet duct (4).

## Description

### Technical Field

The present invention relates to a blood collection device for an extracorporeal blood circuit.

### Background Art

As is well known, in certain surgical operations, during which the functions of the patient's heart are temporarily suspended, extracorporeal blood circuits are carried out using so-called "heart-lung" machines.

Heart-lung machines comprise a series of devices, including a filtering device (also known as "venous reservoir") adapted to filter the incoming blood from the patient, a heat exchanger adapted to regulate the temperature of the blood exiting the filtering device, and an oxygenator adapted to provide the correct supply of oxygen to the blood to be pumped back into the patient. In particular, the blood arriving from the patient is sent to the oxygenator by means of a relevant pumping unit.

The extracorporeal blood circuits comprise, then, a blood collection device coming from a line connected to a vein of the patient and also connected, for possible integrations, to a container, called cardiotome, which collects and filters the blood coming from the operating field. This device is generally referred to as "venous bag".

The collection devices of known type generally comprise a bag defining a blood containment volume and with which the following elements are associated: a blood inlet duct, a blood outlet duct and one or more air exhaust ducts. One of the functions of these venous blood collection devices is to allow the removal of the air present in the collected blood, in order to avoid the same being subsequently infused into the extracorporeal circuit, with possible serious consequences for the patient.

The ability to remove the air present in the blood collected inside the bag is generally a function of the geometry of the bag itself and of the fluid dynamics associated therewith.

However, it frequently happens that the air removal ability of the bags of known type, also known as de-bubbling, is not satisfactory and that part of it therefore escapes through the second duct towards the extracorporeal circuit. In addition, the removal of micro-bubbles in the collection devices of known type is particularly difficult.

### Description of the Invention

The main aim of the present invention is to devise a blood collection device for an extracorporeal blood circuit that allows optimizing, with respect to the devices known to date, the removal of the air present in the blood that is conveyed and collected inside the relevant bag.

Within this aim, one object of the present invention is to improve the air removal ability, with respect to the solutions of known type, also with reference to the micro-bubbles present in the blood.

The objects set out above are achieved by the present blood collection device for an extracorporeal blood circuit according to claim 1.

### Brief Description of the Drawings

Other characteristics and advantages of the present invention will become more apparent from the description of a preferred, but not exclusive, embodiment of a blood collection device for an extracorporeal blood circuit, illustrated by way of an indicative, yet non-limiting example, in the accompanying tables of drawings wherein:
Figure 1 is a front elevation view of a device according to the invention in a first embodiment;
Figure 2 is a front elevation view of a device according to the invention in a second embodiment.

### Embodiments of the Invention

With particular reference to these figures, reference numeral 1 globally indicates a blood collection device for an extracorporeal blood circuit.

The device 1 comprises at least one bag 2 made of a flexible material and defining a containment volume 3 of the blood, wherein said containment volume 3 is delimited by a plurality of perimeter edges 3a,3b,3c. More particularly, the bag 2 comprises two sheets 2a made of a flexible material and mutually associated along the perimeter edges 3a,3b,3c, so as to define the containment volume 3.

The device 1 also comprises at least one inlet duct 4 of the blood at the inlet of the containment volume 3, at least one outlet duct 5 of the blood from the containment volume 3, and at least one air exhaust duct 6 to the outside of the containment volume 3, wherein each of said ducts 4, 5, 6 is associated with the bag 2.

The inlet duct 4, the outlet duct 5 and the exhaust duct 6 define a blood inlet port 4a, a blood outlet port 5a and an air exhaust port 6a, respectively, communicating with the containment volume 3.

The inlet duct 4 and the outlet duct 5 are associated with the bag 2 at a first zone 14 and at a second zone 15, respectively, defined in the lower portion of the bag itself, and the exhaust duct 6 is associated with the bag 2 at a third zone 16 defined in its upper portion.

The terms "upper" and "lower" used herein relate to the position in which the bag is commonly arranged during its use.

According to the invention, the exhaust duct 6 is substantially superimposed on the inlet duct 4, i.e., the inlet duct 4 and the exhaust duct 6 are arranged substantially facing each other, wherein the term "substantially" as used herein means that such ducts may also be slightly offset from each other, but are still arranged on the same side with respect to a longitudinal median plane of the bag 2.

Also according to the invention, the containment volume 3 has a maximum section, measured transversely to the longitudinal axis of the ducts 4, 5, 6, in the proximity of the inlet port 4a.

More particularly, the cross section is measured, again considering the position in which the bag 2 is placed during its use, along a plane of substantially horizontal section.

This allows for a sharp decrease in the velocity of blood inside the containment volume 3 as it flows through the inlet port 4a, which facilitates the upward displacement of the air bubbles and, consequently, their escape through the exhaust duct 6.

Advantageously, the containment volume 3 is delimited by at least a first lateral edge 3a which defines a concavity 13 facing the inside of the containment volume itself. In other words, the first lateral edge 3a is shaped so as to define an outwardly facing "overhang".

Preferably, the first lateral edge 3a has an at least partly curved extension.

More specifically, the first lateral edge 3a connects the first zone 14 to the second zone 15.

Appropriately, the second zone 15 is positioned at a lower level than the first zone 14, just as the outlet port 5a is positioned at a lower level than the inlet port 4a.

As visible from the figures, the bag 2 also comprises at least one substantially curved lower edge 3b, also partly delimiting the containment volume 3, and that connects the first zone 14 to the second zone 15.

More particularly, the bag 2 has, then, a second lateral edge 3c positioned at the second zone 15, on the opposite side of the first lateral edge 3a, where the second lateral edge 3c and a section of the lower edge 3b define a collecting post 17 of the operating liquid positioned on the top of the outlet port 5a.

Advantageously, the bag 2 is shaped in such a way that when the volume of blood contained in the containment volume 3 is substantially equal to 100 ml, the height of the blood contained in the collecting post 17 is greater than 90 mm.

Preferably, when the volume of blood contained in the containment volume 3 is substantially equal to 100 ml, the height of the blood contained in the collecting post 17 is greater than 100 mm.

Additionally, when the volume of blood contained in the containment volume 3 is substantially equal to 200 ml, the height of the blood contained in the collecting post 17, and thus encumbering the outlet port 5a, is comprised between 125 mm and 135 mm.

Thus, the collecting post 17 defines an abrupt shrinkage of the cross section with respect to the maximum cross section.

This is because the greater the height of the blood head positioned on top of the outlet port 5a, the volume being equal, the less macro- and micro-embolic activity, i.e., the amount of air escaping through the outlet port 5a.

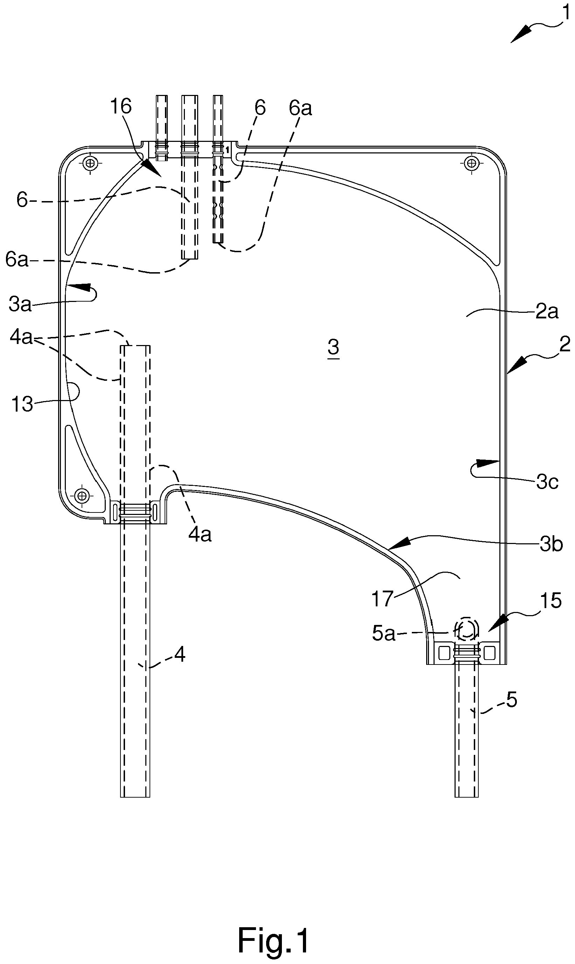

In the embodiment of Figure 1, the inlet port 4a is shaped in such a way to allow the blood to enter the containment volume 3 with an angle substantially equal to 180°.

More in detail, in this embodiment, the inlet port 4a is defined both along a portion of the side wall delimiting the inlet duct 4, by its entire perimeter extension, and at its longitudinal end. In other words, the inlet duct 4 has a plurality of openings positioned on its lateral wall, along its entire perimeter extension, and an opening positioned at its longitudinal end, which result in the outflow of the blood with an angle equal to about 180°.

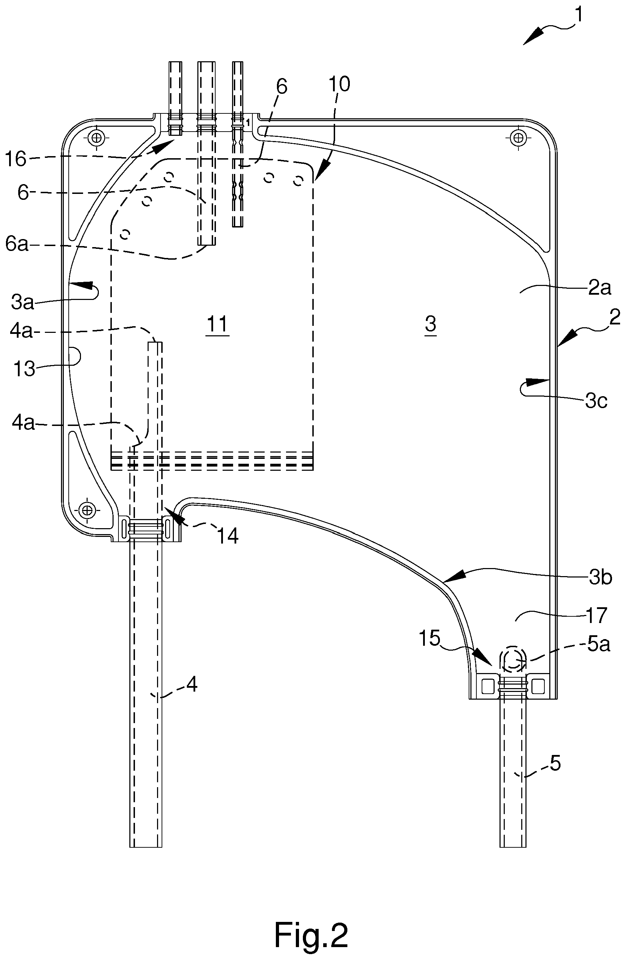

In the embodiment of Figure 2, however, the inlet port 4a is shaped in such a way to allow the blood to enter the containment volume 3 with an angle substantially equal to 90°.

More particularly, in this embodiment, the inlet port 4a is defined both along a portion of the side wall delimiting the inlet duct 4, by a delimited section of its perimeter extension, and at its longitudinal end. In other words, the inlet duct 4 is open at its longitudinal end and at a delimited section, both longitudinally and transversely, of its side wall.

Conveniently, the delimited section of the side wall of the inlet duct, which partly defines the inlet port 4a, faces the concavity 13, so that part of the blood flow entering the containment volume 3 is pushed towards the concavity itself.

In the embodiment of Figure 2, the device 1 also comprises de-bubbling means 10 of the air contained in the operating liquid positioned inside the containment volume 3.

In more detail, the de-bubbling means 10 define a de-bubbling chamber 11, the walls of which are permeable to air and liquids, inside which both the inlet port 4a and the exhaust port 6a are located.

The de-bubbling means 10 are, e.g., composed of a micro-perforated mesh adapted to facilitate the disposal, through the exhaust duct 6, of the air, and in particular of the micro-bubbles, present in the operating liquid entering the containment volume 3.

The operation of the present invention is as follows.

The blood coming from the patient, or from the operating field, is conveyed into the containment volume 3 through the inlet duct 4.

After the blood has entered the containment volume 3, by exiting the inlet port 4a, it undergoes a sharp decrease in velocity due to the abrupt change in section that occurs at this passage.

This decrease in velocity, as discussed above, facilitates the relevant displacement of the air bubbles with respect to the blood, which then move upwards, coming out of the exhaust duct.

In the second embodiment shown in Figure 2, the blood flowing out of the inlet port 4a is initially contained inside the de-bubbling chamber 11 in order to facilitate the escape of air, and in particular of the micro-bubbles, through the exhaust port 6a.

The blood collects, in turn, in the collecting post 17 positioned on top of the outlet port 5a so as to flow out through it.

It has in practice been ascertained that the described invention achieves the intended objects and, in particular, the fact is emphasized that the device to which the present invention relates allows, thanks to its shape and the consequent fluid dynamics of the blood, obtaining an effective removal of the air contained in the blood.

This effect is due in particular to the abrupt slowing down that the blood undergoes when exiting the inlet port and is caused by the rapid change in section to which it is subjected as well as by the positioning of the exhaust duct substantially facing the inlet duct.

In addition, the presence of the collecting post defined at the blood outlet port allows maximizing the height of the blood head, the minimum operating volume being equal.

## Claims

1. Blood collection device (1) for an extracorporeal blood circuit comprising:
- at least one bag (2) made of a flexible material defining a containment volume (3) of the blood, said containment volume (3) being delimited by a plurality of perimeter edges (3a,3b,3c);
- at least one inlet duct (4) of the blood associated with said bag (2) and defining an inlet port (4a) communicating with said containment volume (3);
- at least one outlet duct (5) of the blood associated with said bag (2) and defining an outlet port (5a) of the blood communicating with said containment volume (3);
- at least one exhaust duct (6) of the air defining an exhaust port (6a) communicating with said containment volume (3);
where said inlet duct (4) and said outlet duct (5) are associated with said bag (2), respectively, at a first zone (14) and at a second zone (15) defined in its lower portion and said exhaust duct (6) is associated with said bag (2) at a third zone (16) defined in its upper portion;
**characterized by** the fact that said exhaust duct (6) is substantially superimposed on said inlet duct (4) and by the fact that said containment volume (3) has a maximum section, measured transversely to the longitudinal axis of said ducts (4, 5, 6), in the proximity of said inlet duct (4).

2. Device (1) according to claim 1, **characterized by** the fact that said bag (2) comprises at least a first lateral edge (3a) which delimits at least part of said containment volume (3) and which defines a concavity (13) facing inside the containment volume itself.

3. Device (1) according to claim 2, **characterized by** the fact that said first lateral edge (3a) has an at least partly curved extension.

4. Device (1) according to claim 2 or 3, **characterized by** the fact that said first lateral edge (3a) connects said first zone (14) to said third zone (16).

5. Device (1) according to one or more of the preceding claims, **characterized by** the fact that said second zone (15) is positioned at a lower level than said first zone (14).

6. Device (1) according to claim 5, **characterized by** the fact that said bag (2) comprises at least one substantially curved lower edge (3b) that connects said first zone (14) to said second zone (15).

7. Device (1) according to claim 6, **characterized by** the fact that said bag (2) has at least a second lateral edge (3c), positioned at said second zone (15), on the opposite side of said first lateral edge (3a), and by the fact that said second lateral edge (3c) and a section of said lower edge (3b) define a collecting post (17) of the operating liquid positioned on the top of said outlet port (5a).

8. Device (1) according to claim 7, **characterized by** the fact that when the volume of blood contained in said containment volume (3) is substantially equal to 100 ml, the height of the blood contained in said collecting post (17) is greater than 90 mm.

9. Device (1) according to claim 8, **characterized by** the fact that when the volume of blood contained in said containment volume (3) is substantially equal to 100 ml, the height of the blood contained in said collecting post (17) is greater than 100 mm.

10. Device (1) according to one or more of the preceding claims, **characterized by** the fact that said inlet port (4a) is shaped in such a way as to allow the blood to enter said containment volume (3) with an angle substantially equal to 180°.

11. Device (1) according to claim 10, **characterized by** the fact that said inlet port (4a) is defined both along a section of the side wall delimiting said inlet duct (4), by its entire perimeter extension, and at its longitudinal end.

12. Device (1) according to one or more of the preceding claims 1 to 8, **characterized by** the fact that said inlet port (4a) is shaped in such a way as to allow the blood to enter said containment volume (3) with an angle substantially equal to 90°.

13. Device (1) according to claim 12, **characterized by** the fact that said inlet port (4a) is defined both along a portion of the side wall delimiting said inlet duct (4), by a delimited section of its perimeter extension, and at its longitudinal end.

14. Device (1) according to claim 13, **characterized by** the fact that said delimited section, partly defining said inlet port (4a), faces said concavity (13).

15. Device (1) according to one or more of the preceding claims, **characterized by** the fact that it comprises de-bubbling means (10) of the air contained in the operating liquid positioned inside said containment volume (3).

16. Device (1) according to claim 15, **characterized by** the fact that said de-bubbling means (10) define a de-bubbling chamber (11), the walls of which are permeable to air and liquids, said inlet port (4a) and said exhaust port (6a) being located inside said de-bubbling chamber (11).
